Europäisches Patentamt

European Patent Office

Office européen des brevets

(11) Numéro de publication : **0 610 147 B1**

# (12) FASCICULE DE BREVET EUROPEEN

(45) Date de publication du fascicule du brevet :
**13.09.95 Bulletin 95/37**

(51) Int. Cl.⁶ : **G01N 33/36**

(21) Numéro de dépôt : **94440006.8**

(22) Date de dépôt : **02.02.94**

(54) **Capteur pour la mesure sans contact des caractéristiques d'un produit linéaire de très grande longueur par rapport à ses autres dimensions, sur une machine de production ou autre.**

(30) Priorité : **03.02.93 FR 9301330**

(43) Date de publication de la demande :
**10.08.94 Bulletin 94/32**

(45) Mention de la délivrance du brevet :
**13.09.95 Bulletin 95/37**

(84) Etats contractants désignés :
**CH DE GB IT LI**

(56) Documents cités :
**EP-A- 0 493 050**
**CH-A- 680 310**
**DE-A- 3 803 353**
**US-A- 4 490 618**
**MELLIAND TEXTILBERICHTE, vol. 73, no. 8, août 1992, Heidelberg (DE); LEUENBERGER, pp. 611-613, XP294573/**
**DATABASE WPI, week 9142, Derwent Publications Ltd., London (GB); AN 91-308590/**

(73) Titulaire : **SUPERBA S.A.**
**13 rue de Pfastatt**
**F-68200 Mulhouse (FR)**

(72) Inventeur : **Durand, Bernard**
**28, rue du Rhin**
**F-68120 Pfastatt (FR)**
Inventeur : **Enderlin, Robert**
**9, rue du Petit Bois**
**F-68790 Morschwiller Le Bas (FR)**
Inventeur : **Henry, Pierre**
**44a, rue des Carrières**
**F-68110 Illzach (FR)**

(74) Mandataire : **Nuss, Pierre et al**
**10, rue Jacques Kablé**
**F-67080 Strasbourg Cédex (FR)**

EP 0 610 147 B1

## Description

La présente invention concerne le domaine du contrôle de produits linéaires de très grande longueur par rapport à leurs autres dimensions, à savoir de produits en forme de fils, de mèches, de rubans ou de tissus et a pour objet un capteur pour la mesure sans contact des caractéristiques morpho-dimensionnelles et dynamiques d'un tel produit sur une machine de production ou autre.

La connaissance des caractéristiques d'un produit en forme de fil, de mèche, de ruban ou de tissu est particulièrement nécessaire, dans le domaine de la filature, pour la vérification de la régularité d'un produit donné, à savoir pour détecter les défauts éventuels dûs, soit à un mauvais fonctionnement de la machine, soit à une défectuosité d'un élément de la machine, soit à une irrégularité ou une non homogénéité de la matière première utilisée elle-même, à savoir du fil, de la mèche, du ruban ou du tissu.

Par ailleurs, dans le domaine du tissage de fils textiles ou autres ou encore dans le domaine du bobinage de fils métalliques, se pose également un problème de connaissance instantanée des caractéristiques desdits fils, en particulier de leur tension et/ou de leur vitesse et/ou de leur torsion et/ou de la régularité.

Cette connaissance des caractéristiques des produits linéaires s'applique particulièrement à tous les secteurs de l'industrie textile, tels que la filature, le filage des fibres synthétiques, le bobinage, l'ourdissage et le tissage.

Actuellement, les mesures des caractéristiques essentielles des produits linéaires de ce type sont généralement effectuées par des moyens mécaniques, électriques ou électromagnétiques par contact, à savoir, pour la vitesse, au moyen d'un mécanisme à roulette relié à un compteur, et, pour la tension, au moyen d'un dispositif à roulette monté sur un bras tendeur relié à un dispositif de tarage.

Ces moyens connus présentent, cependant, l'inconvénient d'être en contact avec le fil, de devoir entraîner une roulette, de faire passer le fil par un chemin détourné sinueux avec une nécessité d'embarrage, ce qui induit des frottements, des contraintes de pliage, de torsion, etc..., et ainsi des tensions parasites supplémentaires sur ledit fil.

En outre, ces moyens connus peuvent entraîner la formation de bourres et de poussières et modifier les caractéristiques du produit par usure et échauffement.

Enfin, il est également possible que, suite à un encrassement de la machine ou des éléments de machine, voire du capteur de mesures lui-même par la bourre et les poussières, il se produise des accrochages avec le produit à mesurer ou à contrôler et/ou des collages de matières par adhérence ou fusion (dans le cas de grandes vitesses), du fait de la matière première constitutive du produit à mesurer ou des produits chimiques annexes pour réaliser le fil, tels que les ensimages par exemple.

Actuellement, par exemple, la mesure de la torsion est uniquement possible par prélèvement et par essais destructifs d'échantillons. Une telle mesure n'est pas possible sur un produit en mouvement. De même, la mesure des caractéristiques fondamentales d'un produit de grande longueur, tel qu'un fil textile, par exemple en mouvement, qui sont la vitesse de défilement et la tension, ne peut être réalisée actuellement qu'en plaçant un capteur en contact avec le produit, tel que signalé plus haut.

Il est également connu d'effectuer des mesures de régularité massique ou d'encombrement, par exemple de fils, au moyen de capteurs capacitifs ou optiques permettant, dans ce cas, d'effectuer des mesures sans contact, soit sur des machines de production, soit sur des machines spécifiques, par exemple des appareils de laboratoire.

On connaît ainsi, par FR-A-2 549 096 un procédé de contrôle automatique de fils textiles et un appareil pour la mise en oeuvre de ce procédé, permettant d'effectuer automatiquement à l'aide d'un seul et même appareil, un ensemble de mesures sur au moins une éprouvette de fil, ces mesures étant conçues pour permettre au moins une détermination du titre de ce fil et une détermination de sa régularité de masse linéique, et/ou au moins une détermination de la torsion et une détermination des propriétés dynamométriques de ce fil. Conformément à ce document, les mesures sont réitérées sur une série d'éprouvettes d'un même fil et les résultats sont enregistrés, puis traités par des moyens informatiques, en vue de leur exploitation statistique et/ou de leur archivage.

D'après ce procédé et l'appareil pour sa mise en oeuvre, la mesure de régularité du fil est réalisée au moyen d'un capteur capicitif comportant un condensateur double comprenant une plaque centrale et deux plaques latérales définissant deux interstices dont l'un est traversé par le fil. Ce procédé et ce dispositif permettent l'obtention d'une information relative à la régularité du fil grâce au capteur capacitif et du titre de ce fil grâce à une balance de précision recueillant ledit fil après mesure de sa régularité.

Par FR-A-2 587 806 il a été proposé un dispositif pour la mesure continue de la masse linéique d'un produit textile se présentant sous forme d'un capteur analogue à celui mis en oeuvre dans le procédé selon FR-A-2 549 096, dans lequel sont prévus des moyens modifiant simultanément l'entrefer des deux capacités (condensateurs) constituant ledit capteur. Ce capteur permet une mesure plus précise que les capteurs antérieurs.

Le capteur capacitif mesure la régularité massique du fil, sachant que la variation de masse dans l'entrefer du capteur capacitif entraîne une variation du diélectrique du condensateur formé par les deux armatures de ce capteur, où le diélectrique est constitué par l'air ambiant et la masse du fil situé entre les armatures.

Il est également connu, par FR-A-2 651 888, un procédé et un appareil permettant la caractérisation et la mesure de la qualité de rubans et de mèches ou de fils de fibres textiles.

Ce procédé et ce dispositif consistent également à mettre en oeuvre des échantillons et à réaliser sur ces derniers des mesures de régularité, de l'allongement de la force de rupture desdits échantillons, ainsi que de la détermination du titre desdits échantillons. Un tel dispositif permet, certes, de caractériser rapidement un grand nombre d'échantillons, mais n'est pas du tout adapté à une mesure en continu, du fait même de sa constitution et des moyens de mesure utilisés. En outre, l'ensemble des capteurs ne permet pas de déterminer la vitesse et les tensions du fil.

De plus, dans les capteurs connus, il est usuel de prévoir un réglage de l'alimentation en fréquence de la ou des capacités en fonction de certains paramètres physico-chimiques, tels que l'humidité.

FR-A-2 657 959 décrit un procédé et un dispositif de mesure de la torsion d'un fil textile, dans lesquels le fil est éclairé par voie optique au moyen d'un faisceau lumineux, afin de former une tache lumineuse correspondant à la lumière diffractée par les fibres de surface de l'âme, cette tache lumineuse étant examinée en vue de l'analyse de la répartition énergétique, afin de définir l'angle de torsion.

Enfin on connaît, par FR-A-2 657 958 un procédé et un dispositif de mesure d'au moins une dimension transversale d'un fil textile, ledit procédé consistant à éclairer le fil avec un faisceau de lumière cohérente, à examiner la figure d'interférence obtenue dans le plan focal d'un système optique et à déduire la dimension transversale de la distance séparant les franges d'interférence, au moins deux franges symétriques étant ménagées par rapport au fil pour former deux sources secondaires en interférence, la dimension transversale du fil étant déduite de l'écart entre les franges de la figure d'interférence obtenue.

Ces procédé et dispositif connus permettent de déterminer notamment le numéro d'un fil, mais, du fait de l'encombrement nécessaire du dispositif et de son montage autour du fil à mesurer, son application à une mesure en continu sur une machine de production ou pour une mesure temporaire à l'entrée ou à la sortie d'une telle machine, n'est pas envisageable.

Si les moyens précités permettent des mesures de régularité et de torsion sans contact et si, par une miniaturisation poussée, la réalisation de ces mesures était permise sans contact sur des machines de production, aucun de ces moyens ne permet des mesures simultanées, sans contact, de la vitesse d'un produit linéaire, de la régularité massique, absolue ou non, de la tension, ni de la torsion.

Par ailleurs, la publication MELLIAND TEXTILBERICHTE vol. 73, n° 8, Août 1992, HEIDELBERG pages 611-613, XP294573 - LEUENBERGER 'Fadenspannungen berührungslos ermitteln' décrit une technique de mesure de la tension d'un fil sur un continu à filer par mesure de la vibration circulaire du fil par rapport à un oeillet de guidage du fil. Cette mesure est particulièrement difficile à réaliser, du fait que le fil suit une trajectoire spatiale de grande amplitude, de sorte que l'adjonction d'un capteur de tension avec embarrage est inconcevable. Le fil est soumis à une vibration circulaire qui fait apparaître un ventre et un noeud sur le fil en formation, de sorte qu'à cet endroit le fil est fragile et que l'installation d'un capteur ne peut y être envisagée.

Les oscillations circulaires du fil sont enregistrées au moyen d'une caméra et le noeud de vibration est déterminé à partir de la courbe enveloppe extraite de l'enregistrement desdites oscillations.

D'après ce document, le fil n'est soumis à aucune vibration extérieure, la vibration étant générée en cours de fonctionnement de la machine. Le capteur regarde la vibration et, après traitement, en déduit la tension. Cependant, ce capteur n'est pas capable d'extraire la composante de vibration d'un fil, même si elle est minime, ceci sans contact et le fil étant en déplacement.

La présente invention a pour but de pallier les inconvénients des capteurs et dispositifs de mesure connus à ce jour, en proposant de créer des capteurs originaux de nouvelle génération. Ces nouveaux capteurs peuvent déterminer simultanément plusieurs ou toutes les caractéristiques citées plus haut, ceci par des mesures directes individuelles ou par des mesures de fonctions partielles, qui, par intercorrélation, permettent de déduire les autres caractéristiques ou paramètres d'un produit linéaire de très grande longueur par rapport à ses autres dimensions, sur une machine de production ou autre.

A cet effet, elle a pour objet un capteur caractérisé en ce qu'il opère sans contact et en ce qu'il est muni d'au moins un moyen présentant des zones de mesure par rapport à une position moyenne du fil en défilement et mesurant la variation du complexe caractéristiques morpho-dimensionnelles et/ou position du fil, cette dernière variant, soit par mouvement vibratoire, soit par déplacement induit de la position moyenne de l'axe de défilement, les zones de mesure présentant chacune une sensibilité différente et variable par rapport au plan médian de l'axe du fil à mesurer.

L'invention sera mieux comprise, grâce à la description ci-après, qui se rapporte à des modes de réalisation préférés, donnés à titres d'exemples non limitatifs, et expliqués avec référence aux dessins schématiques annexés, dans lesquels :

la figure 1 est une vue en élévation et/ou en coupe par un plan médian d'un capteur conforme à l'invention ;

la figure 2 est une vue analogue à celle de la figure 1 d'une variante de réalisation de l'invention ;

la figure 3 est une vue en élévation latérale du capteur suivant la figure 2 ;

les figures 4 à 10 sont des vues analogues à celles de la figure 2 de différentes variantes de réalisation du capteur ;

les figures 11 et 12 sont des vues analogues à celle de la figure 3 de variantes de réalisation de la géométrie transversale des capteurs ;

la figure 13 est une vue en coupe d'un capteur circulaire et/ou tubulaire ;

la figure 14 est une vue en perspective d'une autre variante de réalisation de l'invention, et

les figures 15 et 16 sont des vues en élévation latérale de capteurs optiques.

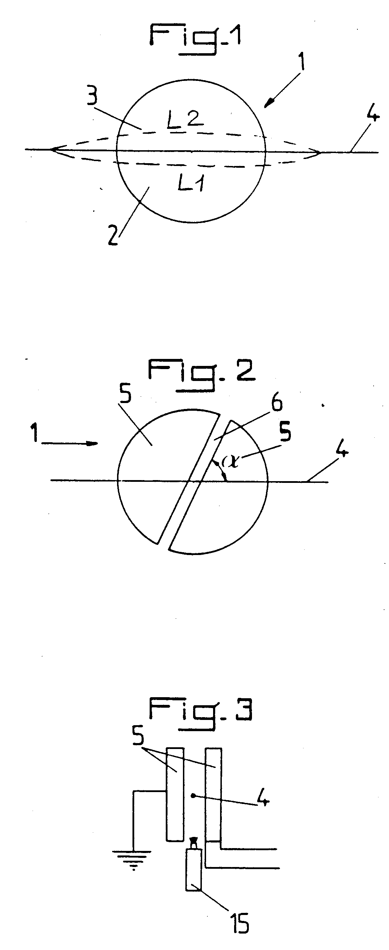

Conformément à l'invention, et comme le montrent plus particulièrement les figures 1 à 16 des dessins annexés, le capteur pour la mesure des caractéristiques d'un produit linéaire de très grande longueur par rapport à ses autres dimensions, sur une machine de production ou autre, opère sans contact et est muni d'au moins un moyen 1 présentant des zones 2 et 3 de mesure par rapport à une position moyenne du fil 4 et mesurant la variation du complexe caractéristiques morpho-dimensionnelles et position du fil 4, cette dernière variant, soit par un mouvement vibratoire, soit par déplacement induit de la position moyenne de l'axe de défilement.

Selon une caractéristique de l'invention, les zones 2 et 3 présentent chacune une sensibilité différente et variable par rapport au plan médian de l'axe du fil à mesurer 4. Il en résulte qu'une vibration du fil à mesurer 4 parallèlement au plan du capteur 1 entraînera l'enregistrement de mesures dans les zones 2 et 3, correspondant à la longueur du fil 4 pendant sa vibration en déplacement et entraînant une variation correspondant à la variation de longueur du fil dans ses positions de vibration dans les zones 2 et 3.

Conformément à une autre caractéristique de l'invention, la sensibilité des zones 2 et 3 peut être variable linéairement ou suivant une autre fonction mathématique, à savoir exponentielle, parabolique ou autre, en fonction d'un traitement de surface ou des constituants des zones 2 et 3 en eux-mêmes. Ainsi, il est possible de munir les zones 2 et 3 d'un revêtement conducteur ou réfléchissant, dont la conduction ou la réflexion varie progressivement d'un bord d'une zone à l'autre bord ou encore influe directement sur la matière et/ou la géométrie des constituants des zones 2 et 3.

Le capteur conforme à l'invention peut être de type capacitif, inductif ou optique. Ainsi, le capteur conforme à l'invention peut être adapté à la mesure de tout type de produit linéaire en défilement, ledit produit agissant comme diélectrique, dans le cas d'utilisation d'un capteur capacitif, et étant par exemple un fil, une mèche, un ruban ou un tissu en un matériau non conducteur. Dans le cas où le produit linéaire est un produit conducteur, le capteur peut être un capteur inductif, les variations des caractéristiques du produit ayant pour conséquence de modifier le champ magnétique entre les pôles du capteur inductif. Enfin, dans le cas où le capteur est sous forme d'un capteur optique, les variations de son éclairement par une source de lumière, cohérente ou non, projetée à partir du côté opposé du capteur ou de la lumière réfléchie provenant d'une source intégrée audit capteur ou éclairant le produit de profil, sont relevées sous forme d'un signal d'intensité.

En présence d'un capteur capacitif, une variation des caractéristiques morpho-dimensionnelles du produit, ainsi qu'une vibration ou un déplacement dudit produit auront pour conséquence immédiate une variation du diélectrique, qui peut être mesurée et enregistrée.

Il en est de même en ce qui concerne un capteur inductif utilisé en relation avec un produit conducteur et dont le champ magnétique varie directement en fonction des modifications de la position suite à la vibration dudit produit. Dans le cas d'utilisation d'un capteur optique, ce sont les variations d'intensité lumineuse qui traduisent les modifications des caractéristiques morpho-dimensionnelles du produit et, notamment, la vibration, et permettent la délivrance d'un signal correspondant.

Selon une caractéristique de l'invention, la sensibilité de la mesure est obtenue par variation de la géométrie de la surface des zones du capteur 1 définies par rapport à la position moyenne du fil 4, de manière linéaire ou non (figures 1 à 16). En effet, comme le montre la figure 1, une vibration du fil à mesurer 4 devant le capteur 1 permettra des relevés de mesures dans la zone à géométrie variable, dans un premier temps dans la zone médiane, puis dans la zone 3, en position haute, puis dans la zone 2, en position basse. Ces mesures prennent en compte des longueurs de fil 4 proportionnelles à la surface des zones 2 et 3 et de la zone médiane du capteur 1 en regard. Dans le cas d'une sensibilité de surface des zones 2 et 3 variable, ces dernières peuvent présenter une géométrie à bords parallèles, à savoir, par exemple, des armatures rectangulaires, la vibration étant détectée sous forme d'un signal variable en fonction de la sensibilité de la zone 2 ou 3 occultée, sans variation de longueur du produit présent entre les armatures.

La figure 1 des dessins annexés représente un premier mode de réalisation de l'invention, dans lequel la surface 1 formant le capteur se présente sous forme d'un disque. Ainsi, une vibration du fil à mesurer 4 par rapport à son axe de défilement permettra de relever un signal différent correspondant à des mesures successives dans les zones médiane et 2 et 3 ou seulement dans une seule zone 2 ou 3 et représentées par L1 et L2 sur la figure 1. Ce signal amplifié conduit à la perception du phénomène vibratoire, qui peut être traduite par un enregistrement de courbes correspondant à une suite de signaux ainsi relevés ou à un traitement d'une suite de signaux en vue d'un contrôle en continu et/ou d'une régulation.

Dans le cas de la mesure de la régularité massique, il apparaît, lors du passage du fil, une perturbation due à la vibration, de sorte que, pour la mesure de la vibration avec un capteur conforme à celui de la figure 1, la sensibilité de ce capteur à la variation de masse entraîne une perturbation importante de la mesure de la vibration.

L'invention a donc également pour objet de créer un capteur indépendant de la variation de la masse et isolant la vibration.

Ce problème peut être résolu par utilisation d'un capteur où le déplacement du fil induit une réponse différentielle sur ledit capteur qui présente à cet effet des éléments dont le comportement est variable en fonction de la position du fil, mais qui sont montés en opposition. A cet effet, il peut être prévu deux éléments de capteur ayant des réponses variables en fonction de la position du fil dans le capteur et qui sont montés en opposition de sensibilité. Ainsi, la mise en rapport des réponses de ces deux éléments n'occasionne une variation que s'il y a déplacement dans un plan perpendiculaire à la trajectoire du fil dans le champ du capteur. La variation de la position deviendra prépondérante par rapport à la réponse correspondant à la variation de masse.

Une telle variation peut être écrite selon une loi de répartition f(R) de la sensibilité du capteur, dans laquelle :

$$R = \frac{P1 \times C1}{P2 \times C2}$$

où :

P1 et P2 : réponses morpho-dimensionnelles prises en compte respectivement dans les zones 1 et 2 du capteur dans l'évolution des propriétés des zones, c'est-à-dire qui varient le long de la longueur perpendiculaire au déplacement du fil des zones,

C1 et C2 : caractéristiques liées au fil ; ces caractéristiques sont proches si les dimensions du capteur sont faibles

f(R) est une loi de réponse des zones le long de la dimension perpendiculaire au déplacement du fil et son évolution peut être logarithmique, sinusoïdale ou exponentielle ; à titre d'exemple, cette loi prend en compte une variation de capacité, d'intensité lumineuse, d'espace de mesure, de profondeur de champ, d'une fréquence vibratoire quelconque, voire d'une polarisation.

Ce type de capteur peut également être utilisé pour analyser des évolutions en position de caractéristiques prélevées sur le fil et, par extension, réaliser une analyse de tous types d'informations en déplacement, sous forme de fil, de lumière réfléchie ou diffuse, etc...

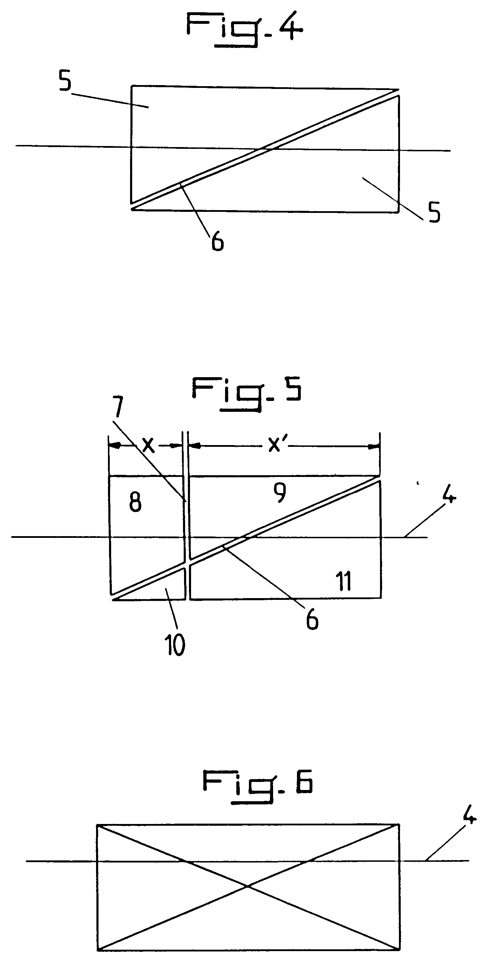

Les figures 2 et 3 des dessins annexés représentent une variante de réalisation du capteur suivant la figure 1, dans laquelle ledit capteur est constitué, dans au moins un plan parallèle au plan médian du fil à mesurer 4, par deux surfaces 5 complémentaires séparées entre-elles par une fente inclinée 6. Ainsi, il est possible d'effectuer une comparaison des mesures relevées sur chaque surface 5 et, du fait que ces surfaces sont complémentaires, d'obtenir une amplification de la variation de mesure correspondant à une vibration. En effet, une vibration du fil 4 engendrera, de part et d'autre de sa position moyenne en défilement, la détection, pour chaque surface 5, d'une longueur donnée, permettant de déterminer pour cesdites surfaces 5 une variation de longueurs et, en effectuant le rapport des valeurs obtenues, une mesure différentielle amplifiée correspondant à une vibration.

Dans ce cas, la sensibilité du capteur est réglable par rotation autour de son axe central. En effet, une rotation de ce capteur autour de son axe central permet une variation de l'inclinaison de la fente séparant ses deux surfaces constituantes, de sorte que les mesures de vibration relevées peuvent être plus ou moins fortement amplifiées, ceci tout en maintenant une constance des dimensions de capteur prises en compte. Ainsi, une variation par rotation de l'angle α entre la fente 6 et l'axe de défilement du fil 4 d'une position perpendiculaire vers une position inclinée dans laquelle l'angle α est très réduit, aura pour effet de faire varier la sensibilité du capteur aux variations de mesure entre une valeur 0 et une valeur de sensibilité très importante, la sensibilité étant faible pour un angle important, proche de 90°, et forte pour un angle de faible valeur.

Ce mode de réalisation permet de s'affranchir des variations morpho-dimensionnelles du produit linéaire 4. Il ne reste donc comme information importante que la position du produit 4 relativement au capteur.

L'obtention d'un signal de vibration permet, par intégration au moyen d'un calculateur d'autres données relatives au fil affichées manuellement ou saisies d'une autre manière, de déterminer la tension de ce dernier par application de la formule simplifiée :

$$P = \frac{\pi^2}{\ell^2} \left[ \sqrt{\frac{E\,I_g}{\gamma\,A}} \right]$$

dans laquelle :

P : fréquence de vibration du produit linéaire
$\ell$ : longueur du produit linéaire entre appuis
E : module d'élasticité du matériau
I : moment d'inertie
g : accélération due à la pesanteur
$\gamma$ : poids spécifique
A : aire de la section du produit linéaire.

Il en résulte qu'en connaissant la longueur du fil dans son parcours de mesure, c'est-à-dire, par exemple, entre la sortie d'une machine et un renvoi vers une machine en aval ou entre deux points d'appui prédéterminés disposés de part et d'autre du capteur, solidaires de ce dernier ou non et réglables en position ou non, sa pulsation ou fréquence de vibration et sa masse linéique déterminée par son numéro, il est possible de déduire la tension du fil en cours de service.

Cette connaissance de la tension est particulièrement importante dans le cadre de la fabrication de fils, du fait que lors de leur transformation les tensions appliquées doivent être parfaitement maîtrisées. Lors du déplacement du fil, il se produit une modification de la fréquence apparente de vibration pour un capteur immobile. Dans le cas d'un produit linéaire ne présentant aucune vibration, en déplacement ou non dans le capteur, il peut être prévu, comme le montre la figure 3, un dispositif d'excitation 15, de type électrostatique, mécanique, pneumatique ou sonore, engendrant une vibration du fil 4 à une fréquence propre donnée par la formule :

$$P = \frac{\pi^2}{\ell^2} \left[ \sqrt{\frac{E\,I_g}{\gamma\,A}} \right]$$

Ainsi, la vibration initialisée dans le fil pourra être mesurée par le capteur.

La longueur sans contact du passage du fil peut facilement être déterminée pour une machine donnée. Cependant, la masse linéique et les variations de masse, de même que la vitesse de passage du fil, peuvent varier, parfois dans une mesure importante, de sorte que la tension déterminée sur la base de la fréquence de vibration peut subir des modifications incompatibles avec une surveillance de fonctionnement correcte.

A cet effet, l'invention propose en variante un capteur permettant de distinguer simultanément les différents paramètres, sans affichage préalable d'aucune donnée.

Ainsi, les figures 4 à 9 représentent des variantes de réalisation des capteurs suivant les figures 2 et 3, dans lesquelles le capteur est constitué par une multiplicité de surfaces permettant chacune une mesure individuelle et un traitement combiné, afin d'aboutir à une caractérisation multiple du produit linéaire en défilement.

La figure 4 représente une variante de réalisation du capteur suivant les figures 2 et 3, dans laquelle le capteur est constitué par deux surfaces symétriques 5 polygonales séparées par une fente inclinée 6.

La variante de réalisation selon la figure 5 correspond au mode de réalisation suivant la figure 4, complété par une séparation du capteur suivant une fente 7 verticale par rapport au produit linéaire en défilement.

Ce capteur présente, de ce fait, quatre surfaces 8 à 11 pouvant être identiques par paires 8, 11 et 9, 10 diagonalement opposées.

Dans la représentation suivant cette figure les surfaces 8 à 11 sont de tailles différentes, et sont séparées par une fente verticale 7 et par une fente diagonale 6.

Ainsi, le capteur permet la distinction, par combinaisons simultanées des mesures relevées au niveau de chaque surface 8 à 11, de la régularité massique, la vibration et la vitesse et, par intégration de ces paramètres au moyen d'un calculateur, la tension selon la formule simplifiée :

EP 0 610 147 B1

$$P = \frac{\pi^2}{\ell^2} \left[ \sqrt{\frac{E\, I_g}{\gamma\, A}} \right]$$

En effet la somme des mesures relevées dans les surfaces 8 à 11 permet un fonctionnement du capteur comme un capteur conventionnel de régularité morpho-dimensionnelle, connu en soi. Ce capteur relatif peut être étalonné, en vue de son utilisation comme capteur absolu, dans le cas où la matière est connue et dans les conditions de mesure. Par exemple, pour un capteur capacitif, une balance permettrait d'établir la constante diélectrique du produit dans les conditions de l'expérimentation.

L'association des paires de mesures relevées de part et d'autre de la diagonale 6, à savoir des mesures relatives aux surfaces 8 + 9 et 10 + 11, permet la détection par effet différentiel de toutes les vibrations du fil. En effet, chaque déplacement latéral du fil 4 dans l'entrefer du capteur induit une augmentation de longueur de fil dans la partie de capteur formée par les surfaces 8 et 9 et une diminution de la longueur de fil détectée dans la partie de capteur constituée par les surfaces 10 et 11.

Par un traitement des signaux de mesure correspondants obtenus dans un demi-pont de Wheatstone, il sera possible de déduire un signal de sortie correspondant à la vibration.

Par l'association des surfaces 8, 10 et 9, 11 ou éventuellement 8, 10 et 8 à 11 ou encore 9, 11 et 8 à 11, les surfaces de longueurs différentes ou non permettront, suivant le cas, une déduction de la vitesse de défilement du fil par une méthode d'analyse utilisant les fonctions d'intercorrélation ou un filtrage des variations d'amplitudes.

Dans le mode de réalisation selon la figure 5, la longueur x des surfaces 8 et 10 est inférieure à la longueur x′ des surfaces 9 et 11 et la vitesse de défilelement peut être déduite d'une combinaison des surfaces 8 et 10 et des surfaces 8 à 11 ou 9 et 11, dans laquelle la somme des mesures des surfaces 8 et 10 sera considérée comme un signal électrique destiné à subir un filtrage par comparaison avec la somme des mesures de l'ensemble des surfaces 8 à 11 ou 9 et 11 agissant comme un filtre modifiant la forme du premier signal.

Par traitement des amplitudes relatives des variations, qui évoluent en fonction de la longueur du champ de mesure, il est possible de déduire la vitesse du fil. En effet, par la construction du capteur on connaît la longueur du champ de mesure, de sorte que le signal émetteur peut être induit.

Grâce à ce mode de réalisation, il est possible d'effectuer un traitement de l'information électrique permettant l'obtention de la vitesse pratiquement en temps réel, ceci en tenant compte de l'évolution des sensibilités des zones 1 et 2, qui peuvent par exemple présenter une sensibilité à évolution linéaire, mais de sens opposé, par exemple en se présentant sous forme de deux triangles rectangles disposés tête-bêche (figure 4), ou encore de sensibilité à évolution suivant une autre loi mathématique, avec une évolution exponentielle, parabolique ou autre.

Dans le cas où les surfaces 8 et 10 et 9 et 11 sont séparées par une médiane verticale 7, c'est-à-dire où x = x′, l'utilisation des fonctions d'intercorrélation entre les mesures relevées au niveau des surfaces 8 et 10 et celles relevées au niveau des surfaces 9 et 11, permettra le calcul de la vitesse, du fait que l'intervalle de temps, qui sépare les signaux électriques identiques émis par les combinaisons des surfaces 8 et 10 et 9 et 11 et la distance séparant les groupes de surfaces sont connus, de sorte qu'il est possible de déduire forcément la vitesse du fil 4.

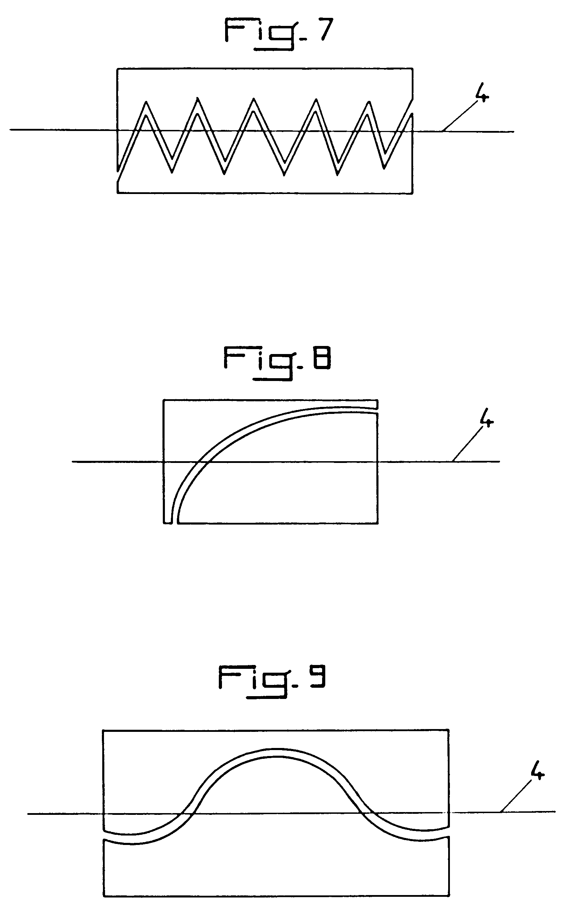

Les figures 6 et 7 représentent des variantes de réalisation de l'invention, dans lesquelles il est possible de relever plus d'une paire de mesures, à savoir suivant la figure 6, au moins trois mesures à chaque pulsation du fil 4 et, suivant la figure 7, une multitude de mesures, les surfaces multiples étant agencées de façon différente, par symétrie ou par des séparations en dents de scie, afin de simplifier ou d'amplifier les signaux recueillis sur les différentes surfaces, en tenant compte de la forme de ces dernières.

La figure 8 des dessins annexés représente une variante de réalisation de l'invention, dans laquelle les surfaces présentent une ligne de séparation de forme parabolique, exponentielle, sinus, cosinus et dérivées. Ainsi, il est possible de réaliser un réglage complémentaire de la sensibilité du capteur par déplacement de l'axe de défilement du fil à mesurer 4 perpendiculairement audit axe de défilement. Il en résulte qu'en fonction de cette disposition, les rapports de longueurs du fil d'une surface à l'autre sont plus ou moins importants et que la variation de la longueur suite à la vibration est plus ou moins amplifiée.

Dans ce mode de réalisation, le traitement des informations est effectué de manière identique à celle décrite à propos de la figure 2.

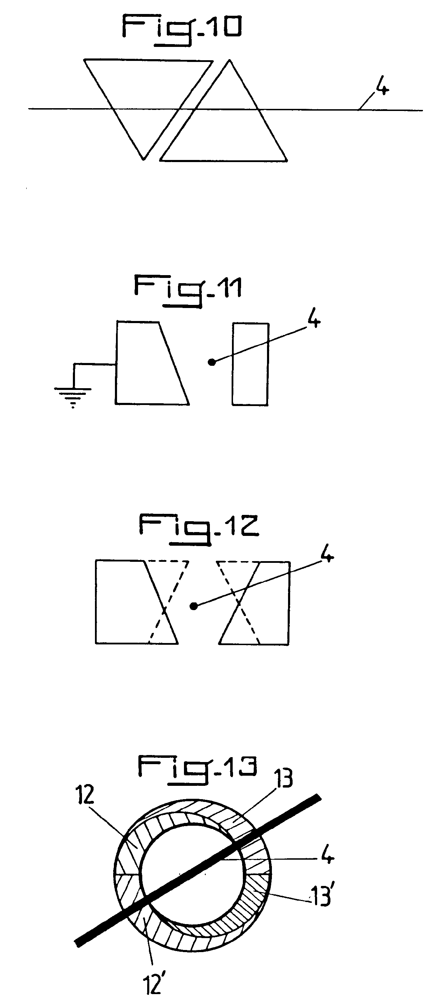

La figure 9 représente une variante de réalisation de la figure 8, dans laquelle les surfaces délimitées par une ligne de séparation de forme parabolique, exponentielle ou autres, sont multipliées, opposées ou symétriques et le traitement des informations s'effectue comme dans le cas du capteur suivant la figure 5. En outre, la sensibilité de ces surfaces peut être variable.

La figure 10 des dessins annexés représente une variante de réalisation du capteur suivant les figures 2 et 3, dans laquelle le capteur est constitué par deux surfaces identiques symétriques par rapport à une diagonale.

Selon une autre caractéristique de l'invention, représentée aux figures 11 et 12, au moins une surface 1 du capteur peut être inclinée par rapport au plan médian du fil à mesurer 4. Il en résulte la possibilité de réaliser une mesure pour tout le plan de la composante de vibration du fil 4. Une telle mesure peut être une mesure simple, suivant le mode de réalisation de la figure 11, ou une mesure différentielle, suivant le mode de réalisation de la figure 12. Dans ce dernier cas, les surfaces 1 sont inclinées symétriquement par rapport au plan médian de l'axe de défilement du fil 4. Par ailleurs, il est également possible de combiner les mesures ainsi obtenues avec celles obtenues par le défilement devant les parties de surfaces successives, ce afin d'augmenter encore la sensibilité.

La figure 14 des dessins annexés représente une autre variante de réalisation de l'invention, dans laquelle au moins deux capteurs sont disposés sur le parcours de fil, ruban, mèche ou tissu avec un décalage en rotation de 90° l'un par rapport à l'autre. Ainsi, l'un des capteurs mesure la composante verticale du produit linéaire considéré et l'autre sa composante horizontale.

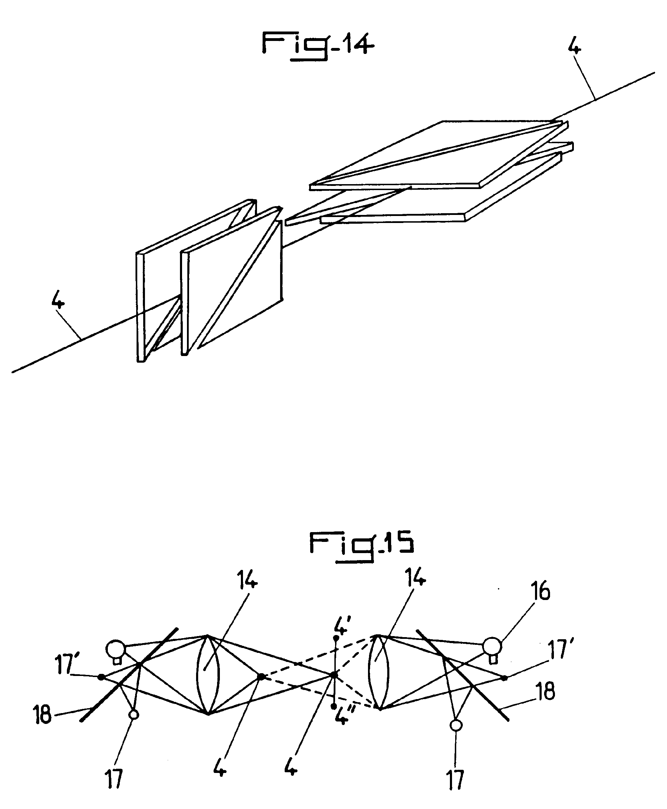

La figure 13 des dessins annexés représente une variante de réalisation de l'invention, dans laquelle le capteur est un capteur circulaire ou semi-circulaire, ou encore tubulaire ou semi-tubulaire, dont les zones sensibles 12, 13 et/ou 12′, 13′ s'étendent en forme de demi-croissants opposés ou de demi-cercles concentriques opposés en sensibilité.

Dans ce mode de réalisation, une déviation du fil par rapport à sa trajectoire entraînera une variation des mesures de longueur relevées dans les zones 12 et 13 et éventuellement 12′ et 13′, ce qui permettra un traitement des données analogue à celui effectué au moyen des capteurs, notamment suivant les figures 4 et 5.

Par ailleurs, une rotation du fil 4 autour de l'axe du capteur aura pour conséquence une prise en compte d'une caractéristique du fil différente induite par l'angle du fil avec le capteur. Cette caractéristique est détectée par les surfaces 12 et 13 et éventuellement par les surfaces 12′ et 13′. Ainsi, le capteur selon la figure 13 permet de tenir compte des modifications intervenant dans les caractéristiques d'un fil ou autre, notamment dans le cas d'un changement de direction de ce dernier autour de l'axe du capteur.

Enfin, la ou les surfaces constituant le capteur conforme à l'invention, peut ou peuvent être pourvue(s), dans le cas d'une réalisation comme capteur optique, de fibres optiques formant récepteurs et/ou sources de lumière cohérente ou non et/ou réflecteurs.

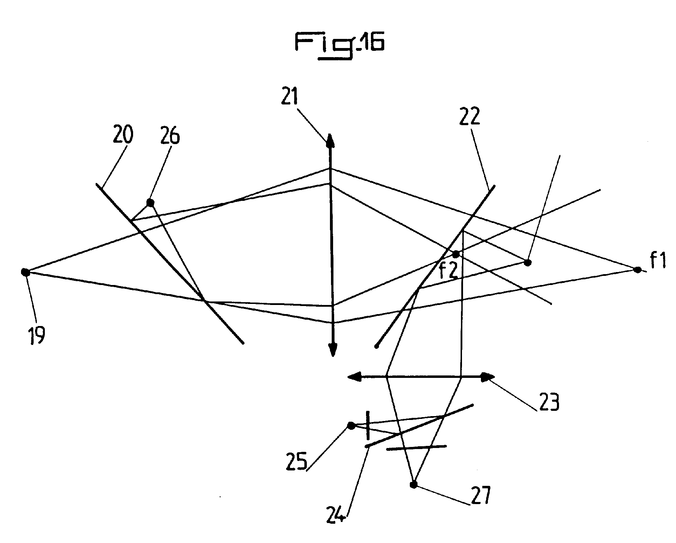

Les figures 15 et 16 des dessins annexés représentent des variantes de réalisation de l'invention, dans lesquelles le capteur est constitué sous forme d'un capteur optique. Ainsi, la figure 15 représente un capteur optique constitué par deux lentilles 14 disposées de part et d'autre du produit linéaire 4 à mesurer, ce produit étant éclairé par une ou deux sources 16 de lumière cohérente ou non et le signal réfléchi étant transféré, sous forme d'une tache lumineuse, derrière la lentille 14 correspondante, par l'intermédiaire d'un miroir semi-réfléchissant 18 à une cellule de mesure 17 correspondante. Ainsi, un déplacement horizontal du produit linéaire 4 entre les lentilles 14 aura pour effet une variation de l'image captée par les cellules 17 et une intercorrélation des signaux relevés par ces cellules permettra de définir la variation du déplacement correspondant à la vibration horizontale du produit 4. Dans le cas d'une variation verticale, les cellules 17 détecteront un déplacement de l'image du fil 4′, 4″, sur leurs parties sensibles.

Selon une variante de réalisation de l'invention, ce capteur peut être complété par deux cellules 17′ disposées dans l'axe des lentilles 14 derrière les miroirs semi-réfléchissants 18. Ces capteurs peuvent notamment détecter directement une vibration du fil 4 dans des positions 4′, 4″ perpendiculairement au déplacement du fil 4 mesuré par les cellules 17.

La figure 16 des dessins annexés représente une autre variante de réalisation d'un capteur optique, dans laquelle ce dernier est constitué par une première source lumineuse 19 disposée derrière un miroir semi-réfléchissant 20 et éclairant à travers ce dernier et une première lentille 21 le produit linéaire 4, dont l'image est réfléchie sur un deuxième miroir semi-réfléchissant 22 disposé entre la première lentille 21 et le produit 4 et transférant ladite image à travers une deuxième lentille 23, par l'intermédiaire d'un troisième miroir semi-réfléchissant 24, sur une première cellule 25 de détection de la vibration horizontale, une deuxième source lumineuse 26 éclairant la face du premier miroir semi-réfléchissant 20 opposée à la première source lumineuse 19 et son flux lumineux étant focalisé par la première lentille 21 et traversant le deuxième miroir semi-réfléchissant 22 pour éclairer le produit 4 suivant un parcours d'ondes différent de celui de la lumière issue de la première source 19, l'image du produit 4 obtenue étant transférée par l'intermédiaire du deuxième miroir semi-réfléchissant 22 à travers la deuxième lentille 23 et le troisième miroir semi-réfléchissant 24 sur une deuxième cellule 27 de détection de la vibration verticale du produit 4. Les sources lumineuses 19 et 26 émettent avantageusement des flux lumineux de longueurs d'ondes respectives $\lambda 1$ et $\lambda 2$ différentes. Les cellules

EP 0 610 147 B1

25 et 27 peuvent avantageusement présenter une configuration comparable à celle du capteur suivant la figure 4.

Le capteur selon la figure 16 permet ainsi d'effectuer, d'une part, une mesure de la vibration horizontale, c'est-à-dire d'un déplacement du produit 4 suivant une direction perpendiculaire à la lentille 21, consistant à relever les variations d'énergie lumineuse réfléchie par le fil différenciée suivant les longueurs d'ondes λ1 et λ2 de focale différente et, d'autre part, une mesure de la vibration verticale, c'est-à-dire d'un déplacement du produit 4 parallèlement à la lentille 21 et consistant en un déplacement du point lumineux réfléchi et transféré la deuxième cellule de détection 27, ce point étant mesuré successivement sur les surfaces constitutives de cette cellule 27, qui pourrait avoir la structure d'un capteur suivant les figures 2 à 5.

L'utilisation du capteur conforme à l'invention permet la mesure en temps réel de plusieurs informations relatives à un produit linéaire en défilement, sans contact avec ce dernier. Les signaux de mesure ainsi obtenus sont traités par un programme modélisant le comportement du fil suivant la formule simplifiée :

$$P = \frac{\pi^2}{\ell^2} \left[ \sqrt{\frac{E\, I_g}{\gamma\, A}} \right]$$

et résidant dans une unité de calcul informatique de type connu, mettant en oeuvre des filtres et des analyses spectrales, afin d'éliminer les données parasites et de délivrer des signaux proportionnels à la tension, à la vitesse et à la régularité, et pouvant être utilisés comme signaux de mesure pour caractériser le produit linéaire et sa situation dynamique comme signaux de mesure, de commande, de régulation ou d'arrêt d'urgence ou d'alarme.

En outre, ce signal peut également être exploité pour l'affichage des données de fonctionnement.

Lorsque le capteur conforme à l'invention est utilisé pour la régulation ou la surveillance, il peut notamment servir, par mise en oeuvre d'un programme approprié, à la modification en cours de fonctionnement de certaines données relatives au produit linéaire, telles que la vitesse et/ou la tension, ce qui est notamment important lors de l'accomplissement de certaines opérations de fabrication, en particulier dans le domaine du filage pour la régulation de la tension lors de l'étirage de la filature et dans le domaine du tissage pour le contrôle de la tension des fils de trames, lors de l'insertion, ainsi que des fils de chaînes, lors de l'ourdissage, de l'encollage ou du tissage.

En outre, l'invention trouve également son application dans le domaine du traitement d'autres éléments linéaires, voire particulaires, notamment dans le bobinage de fils métalliques.

Grâce à l'invention, il est possible de réaliser des capteurs de type capacitif, inductif, optique, phonique ou ultra-sonique, permettant simultanément la mesure sans contact de la régularité du fil, ainsi que de la vitesse, de la vibration et/ou de la torsion, de sorte que la tension du fil peut être déduite par calcul.

Enfin, le capteur conforme à l'invention peut, en fonction des principes utilisés, avantageusement ne pas être constitué par des parties de mesure ou parties actives décalées, mais par des parties superposées, l'analyse différenciant alors les réponses relevées.

Bien entendu, l'invention n'est pas limitée aux modes de réalisation décrits et représentés aux dessins annexés. Des modifications restent possibles, notamment du point de vue de la constitution des divers éléments, ou par substitution d'équivalents techniques, sans sortir pour autant du domaine de protection de l'invention.

**Revendications**

1. Capteur pour la mesure des caractéristiques d'un produit linéaire de très grande longueur par rapport à ses autres dimensions, sur une machine de production ou autre, caractérisé en ce qu'il opère sans contact et en ce qu'il est muni d'au moins un moyen (1) présentant des zones (2 et 3) de mesure par rapport à une position moyenne du fil (4) en défilement et mesurant la variation du complexe caractéristiques morpho-dimensionnelles et/ou position du fil (4), cette dernière variant, soit par mouvement vibratoire, soit par déplacement induit de la position moyenne de l'axe de défilement, les zones de mesure (2 et 3) présentant chacune une sensibilité différente et variable par rapport au plan médian de l'axe du fil à mesurer (4).

2. Capteur, suivant la revendication 1, caractérisé en ce que la sensibilité des zones de mesure (2 et 3) est

9

variable linéairement ou suivant une autre fonction mathématique, exponentielle, parabolique, en fonction d'un traitement de surface ou des constituants des zones (2 et 3) en eux-mêmes.

3. Capteur, suivant l'une quelconque des revendications 1 et 2, caractérisé en ce que les zones de mesure (2 et 3) sont pourvues d'un revêtement conducteur ou réfléchissant, dont la conduction ou la réflexion varie progressivement d'un bord d'une zone à l'autre bord.

4. Capteur, suivant l'une quelconque des revendications 1 et 2, caractérisé en ce que la sensibilité des zones de mesure (2 et 3) est variable par modification directe de la matière et/ou de la géométrie des constituants desdites zones (2 et 3).

5. Capteur, suivant l'une quelconque des revendications 1 à 4, caractérisé en ce qu'il est de type capacitif, inductif ou optique.

6. Capteur, suivant l'une quelconque des revendications 1 à 5, caractérisé en ce que la sensibilité des zones de mesure (2 et 3) est obtenue par variation de la géométrie de la surface desdites zones définies par rapport à la position moyenne du fil (4), de manière linéaire ou non.

7. Capteur, suivant l'une quelconque des revendications 1 à 6, caractérisé en ce que sa surface se présente sous forme d'un disque.

8. Capteur, suivant l'une quelconque des revendications 1 à 7, caractérisé en ce qu'il est constitué, dans au moins un plan parallèle au plan médian du fil à mesurer (4), par deux surfaces (5) complémentaires séparées entre-elles par une fente inclinée (6).

9. Capteur, suivant la revendication 8, caractérisé en ce que sa sensibilité est réglable par rotation autour de son axe central.

10. Capteur, suivant l'une quelconque des revendications 1 à 9, caractérisé en ce qu'il est muni d'un dispositif d'excitation (15), de type électrostatique, mécanique, pneumatique ou sonore, engendrant une vibration du fil (4) à une fréquence propre donnée.

11. Capteur, suivant l'une quelconque des revendications 1 à 6, caractérisé en ce qu'il est constitué par une multiplicité de surfaces permettant chacune une mesure individuelle et un traitement combiné, afin d'aboutir à une caractérisation multiple du produit linéaire en défilement.

12. Capteur, suivant l'une quelconque des revendications 1 à 6, caractérisé en ce qu'il est constitué par deux surfaces symétriques (5) polygonales séparées par une fente inclinée (6).

13. Capteur, suivant la revendication 11, caractérisé en ce qu'il est constitué, dans au moins un plan parallèle au plan médian du fil à mesurer (4), par deux surfaces complémentaires séparées entre-elles par une fente inclinée et par une fente (7) verticale par rapport au produit linéaire en défilement (4), de manière à former quatre surfaces (8 à 11) pouvant être identiques par paires (8, 11 et 9, 10) diagonalement opposées.

14. Capteur, suivant la revendication 11, caractérisé en ce qu'il est constitué par au moins deux surfaces présentant une ligne de séparation de forme parabolique, exponentielle, sinus, cosinus et dérivées.

15. Capteur, suivant la revendication 14, caractérisé en ce que les surfaces sont multipliées, opposées ou symétriques.

16. Capteur, suivant l'une quelconque des revendications 1 à 6 et 8 à 12, caractérisé en ce qu'il est constitué par deux surfaces identiques symétriques par rapport à une diagonale.

17. Capteur, suivant l'une quelconque des revendications 1 à 16, caractérisé en ce qu'au moins une surface (1) est inclinée par rapport au plan médian du fil à mesurer (4).

18. Capteur, suivant la revendication 17, caractérisé en ce qu'il comporte des surfaces (1) inclinées symétriquement par rapport au plan médian de l'axe de défilement du fil (4).

19. Capteur, suivant l'une quelconque des revendications 1 à 18, caractérisé en ce qu'il est combiné avec au moins un deuxième capteur, les deux capteurs étant disposés sur le parcours du fil, ruban, mèche ou tissu avec un décalage en rotation de 90° l'un par rapport à l'autre.

20. Capteur, suivant l'une quelconque des revendications 1 à 6, caractérisé en ce qu'il est circulaire ou semi-circulaire, ou encore tubulaire ou semi-tubulaire et présente des zones sensibles (12, 13 et/ou 12', 13') s'étendant en forme de demi-croissants opposés ou de demi-cercles concentriques opposés en sensibilité.

21. Capteur, suivant l'une quelconque des revendications 1 à 6 et 20, caractérisé en ce que sa ou ses surface(s) constituante(s) est ou sont pourvue(s) de fibres optiques formant récepteurs et/ou sources de lumière cohérente ou non et/ou réflecteurs.

22. Capteur, suivant l'une quelconque des revendications 1 à 6 et 20, caractérisé en ce qu'il est sous forme d'un capteur optique constitué par deux lentilles (14) disposées de part et d'autre du produit linéaire (4) à mesurer, ce produit étant éclairé par une ou deux sources (16) de lumière cohérente ou non et le signal réfléchi étant transféré, sous forme d'une tache lumineuse, derrière la lentille (14) correspondante, par l'intermédiaire d'un miroir semi-réfléchissant (18) à une cellule de mesure (17) correspondante.

23. Capteur, suivant la revendication 22, caractérisé en ce qu'il est complété par deux cellules (17') disposées dans l'axe des lentilles (14) derrière les miroirs semi-réfléchissants (18).

24. Capteur, suivant l'une quelconque des revendications 1 à 6 et 20, caractérisé en ce qu'il est sous forme d'un capteur optique constitué par une première source lumineuse (19) disposée derrière un premier miroir semi-réfléchissant (20) et éclairant à travers ce dernier et une première lentille (21) le produit linéaire (4), dont l'image est réfléchie sur un deuxième miroir semi-réfléchissant (22) disposé entre la première lentille (21) et le produit (4) et transférant ladite image à travers une deuxième lentille (23), par l'intermédiaire d'un troisième miroir semi-réfléchissant (24) sur une première cellule (25) de détection de la vibration horizontale, une deuxième source lumineuse (26) éclairant la face du premier miroir semi-réfléchissant (20) opposée à la première source lumineuse (19) et son flux lumineux étant focalisé par la première lentille (21) et traversant le deuxième miroir semi-réfléchissant (22) pour éclairer le produit (4) suivant un parcours d'ondes différent de celui de la lumière issue de la première source lumineuse (19), l'image du produit (4) obtenue étant transférée par l'intermédiaire du deuxième miroir semi-réfléchissant (22) à travers la deuxième lentille (23) et le troisième miroir semi-réfléchissant (24) sur une deuxième cellule (27) de détection de la vibration verticale du produit (4).

25. Capteur, suivant la revendication 24, caractérisé en ce que les sources lumineuses (19 et 26) émettent avantageusement des flux lumineux de longueurs d'ondes respectives différentes.


**Patentansprüche**

1. Sensor zur Messung der Eigenschaften eines linearen Produktes großer Länge im Vergleich zu seinen anderen Dimensionen, in einer Produktionsmaschine oder ähnlichem, dadurch gekennzeichnet, daß er kontaktlos arbeitet und mit mindestens einer Vorrichtung (1) versehen ist, die Meßzonen (2 und 3) hinsichtlich einer Mittelposition des Fadens (4) im Vorbeilauf aufweist und die Veränderung des Komplexes Formabmessungseigenschaften und/oder Position des Fadens (4) mißt, wobei letztere entweder durch Schwingungsbewegung oder durch induzierte Versetzung von der Mittelposition der Vorbeilaufachse variiert, wobei die Meßzonen (2 und 3) eine jeweils unterschiedliche und hinsichtlich der Mittelebene der Achse des zu messenden Fadens (4) veränderliche Empfindlichkeit aufweisen.

2. Sensor nach Anspruch 1, dadurch gekennzeichnet, daß die Empfindlichkeit der Meßzonen (2 und 3) linear oder gemäß einer anderen mathematischen Funktion, Exponentialfunktion, Parabelfunktion, als Funktion einer Behandlung der Oberfläche oder der Bestandteile der Zonen (2 und 3) in sich variabel ist.

3. Sensor nach einem der Ansprüche 1 und 2, dadurch gekennzeichnet, daß die Meßzonen (2 und 3) mit einem leitenden oder reflektierenden Belag versehen sind, dessen Leitung oder Reflexion sich von einem Rand einer Zone zum anderen Rand progressiv ändert.

4. Sensor nach einem der Ansprüche 1 und 2, dadurch gekennzeichnet, daß die Empfindlichkeit der Meßzonen (2 und 3) durch direkte Abwandlung des Stoffes und/oder der Geometrie der Bestandteile der Zonen (2 und 3) veränderlich ist.

5. Sensor nach einem der Ansprüche 1 bis 4, dadurch gekennzeichnet, daß es sich um einen kapazitiven, induktiven oder optischen Sensor handelt.

6. Sensor nach einem der Ansprüche 1 bis 5, dadurch gekennzeichnet, daß die Empfindlichkeit der Meßzonen (2 und 3) durch Veränderung der Geometrie der Oberfläche der Zonen erhalten wird, die im Vergleich zu der Mittelposition des Fadens (4) auf lineare oder nichtlineare Weise bestimmt sind.

7. Sensor nach einem der Ansprüche 1 bis 6, dadurch gekennzeichnet, daß seine Oberfläche in Form einer Scheibe ausgebildet ist.

8. Sensor nach einem der Ansprüche 1 bis 7, dadurch gekennzeichnet, daß er in zumindest einer Parallelebene zur Mittelebene des zu messenden Fadens (4) aus zwei komplementären Flächen (5) besteht, die durch einen schräg verlaufenden Spalt (6) voneinander getrennt sind.

9. Sensor nach Anspruch 8, dadurch gekennzeichnet, daß die Empfindlichkeit durch Drehung um seine Mittelachse einstellbar ist.

10. Sensor nach einem der Ansprüche 1 bis 9, dadurch gekennzeichnet, daß er mit einer Erregungsvorrichtung (15) des elektrostatischen, mechanischen, pneumatischen oder akustischen Typs versehen ist, die eine Schwingung des Fadens (4) in einer vorbestimmten geeigneten Frequenz erzeugt.

11. Sensor nach einem der Ansprüche 1 bis 6, dadurch gekennzeichnet, daß er aus einer Vielzahl von Flächen besteht, die jeweils eine individuelle Messung und eine kombinierte Verarbeitung erlauben, um eine vielfältige Bestimmung des linearen Produktes im Vorbeilauf zu erreichen.

12. Sensor nach einem der Ansprüche 1 bis 6, dadurch gekennzeichnet, daß er aus zwei symmetrischen, vieleckigen Flächen (5) besteht, die durch einen schräg verlaufenden Spalt (6) getrennt sind.

13. Sensor nach Anspruch 11, dadurch gekennzeichnet, daß er in zumindest einer Parallelebene zur Mittelebene des zu messenden Fadens (4) aus zwei komplementären Flächen besteht, die durch einen schräg verlaufenden Spalt und einen bezüglich des vorbeilaufenden linearen Produktes (4) vertikalen Spalt (7) voneinander getrennt sind, so daß sie vier Flächen (8 bis 11) bilden, die paarweise (8, 11 und 9, 10) diagonal gegenüberliegend gleich groß sein können.

14. Sensor nach Anspruch 11, dadurch gekennzeichnet, daß er aus mindestens zwei Flächen besteht, die eine Trennungslinie in Form einer Parabel-, Exponential-, Sinus-, Cosinus-Kurve oder dergleichen aufweist.

15. Sensor nach Anspruch 14, dadurch gekennzeichnet, daß die Flächen vervielfacht, gegenüberliegend oder symmetrisch sind.

16. Sensor nach einem der Ansprüche 1 bis 6 und 8 bis 12, dadurch gekennzeichnet, daß er aus zwei identischen, zu einer Diagonale symmetrischen Flächen besteht.

17. Sensor nach einem der Ansprüche 1 bis 16, dadurch gekennzeichnet, daß zumindest eine Fläche (1) bezüglich der Mittelebene des zu messenden Fadens (4) geneigt ist.

18. Sensor nach Anspruch 17, dadurch gekennzeichnet, daß er Flächen (1) aufweist, die bezüglich der Mittelebene der Vorbeilaufachse des Fadens (4) symmetrisch geneigt sind.

19. Sensor nach einem der Ansprüche 1 bis 18, dadurch gekennzeichnet, daß er mit zumindest einem zweiten Sensor verbunden ist, wobei die beiden Sensoren auf der Laufstrecke des Fadens, des Bandes, der Schnur oder Kette mit einer Rotationsversetzung von 90° zueinander angeordnet sind.

20. Sensor nach einem der Ansprüche 1 bis 6, dadurch gekennzeichnet, daß er kreisförmig oder halbkreisförmig oder auch röhrenförmig oder halbröhrenförmig ausgebildet ist und Abtastzonen (12, 13 und/oder 12′, 13′) aufweist, die sich in Form von entgegengesetzten Halbhörnchen oder konzentrischen, in der Empfindlichkeit entgegengesetzten Halbkreisen erstrecken.

21. Sensor nach einem der Ansprüche 1 bis 6 und 20, dadurch gekennzeichnet, daß seine Bestandteilsfläche(n) mit optischen Fasern versehen ist oder sind, die Empfänger und/oder Quellen kohärenten oder nicht-kohärenten Lichtes und/oder Reflektoren bilden.

22. Sensor nach einem der Ansprüche 1 bis 6 und 20, dadurch gekennzeichnet, daß er als optischer Sensor mit zwei Linsen (14) ausgebildet ist, die beiderseits des zu messenden linearen Produktes (4) angeordnet sind, wobei das Produkt von einer oder zwei Quellen (16) kohärenten oder nichtkohärenten Lichtes beleuchtet wird und das reflektierte Signal unter Zwischenschaltung eines halbreflektierenden Spiegels (18) in Form eines Lichtflecks hinter die entsprechende Linse (14) zu einer entsprechenden Meßzelle (17) übertragen wird.

23. Sensor nach Anspruch 22, dadurch gekennzeichnet, daß er durch zwei Zellen (17′) ergänzt ist, die in der Achse der Linsen (14) hinter den halbreflektierenden Spiegeln (18) angeordnet sind.

24. Sensor nach einem der Ansprüche 1 bis 6 und 20, dadurch gekennzeichnet, daß er als optischer Sensor ausgebildet ist, bestehend aus einer ersten Lichtquelle (19), die hinter einem ersten halbreflektierenden Spiegel (20) angeordnet ist und durch letzteren und eine erste Linse (21) hindurch das lineare Produkt (4) beleuchtet, dessen Bild auf einen zweiten halbreflektierenden Spiegel (22) geworfen wird, der zwischen der ersten Linse (21) und dem Produkt (4) angeordnet ist, und das Bild über einen dritten halbreflektierenden Spiegel (24) durch eine zweite Linse (23) hindurch auf eine erste Zelle (25) zur Erfassung der horizontalen Schwingung überträgt, wobei eine zweite Lichtquelle (26) die von der ersten Lichtquelle (19) abgewandte Seite des ersten halbreflektierenden Spiegels (20) beleuchtet und ihr Lichtstrahl von der ersten Linse (21) gebündelt wird und durch den zweiten halbreflektierenden Spiegel (22) hindurchtritt, um das Produkt (4) gemäß einem Wellenverlauf zu beleuchten, der anders ist als derjenige des Lichtes, das von der ersten Lichtquelle (19) abgegeben wird, wobei das erhaltene Bild des Produktes (4) über den zweiten halbreflektierenden Spiegel (22) durch die zweite Linse (23) und den dritten halbreflektierenden Spiegel (24) hindurch auf eine zweite Zelle (27) zur Erfassung der vertikalen Schwingung des Produktes (4) übertragen wird.

25. Sensor nach Anspruch 24, dadurch gekennzeichnet, daß die Lichtquellen (19 und 26) bevorzugt Lichtstrahlen mit jeweils unterschiedlicher Wellenlänge aussenden.

## Claims

1. Sensor for measuring the characteristics of a linear article of very great length compared with its other dimensions, on a production machine or other machine, characterised in that it operates without contact and in that it has at least one means (1) having zones (2 and 3) for measuring with respect to a mean position of the moving thread (4) and measuring the variation in the complex consisting of the morpho-dimensional characteristics and/or position of the wire (4), the latter varying either through vibratory movement or through induced movement of the mean position of the axis of movement, the measurement zones (2 and 3) each having a different sensitivity which is variable with respect to the mid-plane of the axis of the thread to be measured (4).

2. Sensor according to Claim 1, characterised in that the sensitivity of the measurement zones (2 and 3) is variable linearly or according to another exponential or parabolic mathematical function, as a function of a treatment of the surface or of the constituents of the zones (2 and 3) in themselves.

3. Sensor according to any one of Claims 1 and 2, characterised in that the measurement zones (2 and 3) are provided with a conductive or reflective coating, the conduction or reflection of which varies progressively from one edge of a zone to the other edge.

4. Sensor according to either one of Claims 1 and 2, characterised in that the sensitivity of the measurement zones (2 and 3) can be varied by direct modification of the material and/or of the geometry of the constituents of the said zones (2 and 3).

5. Sensor according to any one of Claims 1 to 4, characterised in that it is of the capacitive, inductive or optical type.

6. Sensor according to any one of Claims 1 to 5, characterised in that the sensitivity of the measurement zones (2 and 3) is obtained by varying the geometry of the surface of the said zones which are defined with respect to the average position of the thread (4), linearly or otherwise.

7. Sensor according to any one of Claims 1 to 6, characterised in that its surface is in the form of a disc.

8. Sensor according to any one of Claims 1 to 7, characterised in that it consists, in at least one plane parallel to the mid-plane of the thread to be measured (4), of two complementary surfaces (5) separated from each other by an inclined slot (6).

9. Sensor according to Claim 8, characterised in that its sensitivity is adjustable by rotation about its centre axis.

10. Sensor according to any one of Claims 1 to 9, characterised in that it has an excitation device (15) of the electrostatic, mechanical, pneumatic or sound type, producing a vibration of the thread (4) at a given natural frequency.

11. Sensor according to any one of Claims 1 to 6, characterised in that it consists of a multiplicity of surfaces each allowing an individual measurement and a combined processing, in order to give a multiple characterisation of the moving linear product.

12. Sensor according to any one of Claims 1 to 6, characterised in that it consists of two polygonal symmetrical surfaces (5) separated by an inclined slot (6).

13. Sensor according to Claim 11, characterised in that it consists, in at least one plane parallel to the mid-plane of the thread to be measured (4), of two complementary surfaces separated from each other by an inclined slot and by a slot (7) which is vertical with respect to the moving linear product (4), so as to form four surfaces (8 to 11), which may be identical in diagonally opposed pairs (8, 11 and 9, 10).

14. Sensor according to Claim 11, characterised in that it consists of at least two surfaces having a separation line of parabolic, exponential, sine or cosine shape or derivatives thereof.

15. Sensor according to Claim 14, characterised in that the surfaces are multiplied, opposite or symmetrical.

16. Sensor according to any one of Claims 1 to 6 and 8 to 12, characterised in that it consists of two identical surfaces which are symmetrical with respect to a diagonal.

17. Sensor according to any one of Claims 1 to 16, characterised in that at least one surface (1) is inclined with respect to the mid-plane of the thread to be measured (4).

18. Sensor according to Claim 17, characterised in that it has surfaces (1) which are inclined symmetrically with respect to the mid-plane of the axis of movement of the thread (4).

19. Sensor according to any one of Claims 1 to 18, characterised in that it is combined with at least a second sensor, the two sensors being disposed on the path of the thread, ribbon, strand or cloth with one rotated through 90° with respect to the other.

20. Sensor according to any one of Claims 1 to 6, characterised in that it is circular or semi-circular, or else tubular or semi-tubular, and has sensitive zones (12, 13 and/or 12′, 13′) extending in the form of opposite half-crescents or concentric semi-circles of opposite sensitivities.

21. Sensor according to any one of Claims 1 to 6 and 20, characterised in that its constituent surface or surfaces has or have optical fibres forming receivers and/or sources of light, which may be coherent or otherwise, and/or reflectors.

22. Sensor according to any one of Claims 1 to 6 and 20, characterised in that it is a form of an optical sensor consisting of two lenses (14) disposed on each side of the linear product (4) to be measured, this product being illuminated by one or two sources (16) of light (coherent or otherwise), and the reflected signal being transferred, in the form of a light spot, behind the corresponding lens (14), by means of a semi-reflective mirror (18), to a corresponding measuring cell (17).

23. Sensor according to Claim 22, characterised in that it is supplemented by two cells (17') disposed in line with the lenses (14) behind the semi-reflective mirrors (18).

24. Sensor according to any one of Claims 1 to 6 and 20, characterised in that it is in the form of an optical sensor consisting of a first light source (19) disposed behind a first semi-reflective mirror (20) and illuminating, through the latter and a first lens (21), the linear product (4), the image of which is reflected onto a second semi-reflective mirror (22) disposed between the first lens (21) and the product (4) and transferring the said image through a second lens (23), by means of a third semi-reflective mirror (24), onto a first cell (25) for detecting the horizontal vibration, a second light source (26) illuminating the face of the first semi-reflective mirror (20) opposite the first light source (19) and its light flux being focused by the first lens (21) and passing through the second semi-reflective mirror (22) so as to illuminate the product (4) in a wave path different from that of the light coming from the first light source (19), the image of the product (4) obtained being transferred by means of the second semi-reflective mirror (22) through the second lens (23) and the third semi-reflective mirror (24) onto a second cell (27) for detecting the vertical vibration of the product (4).

25. Sensor according to Claim 24, characterised in that the light sources (19 and 26) advantageously emit light fluxes of different respective wavelengths.

Fig.1

Fig. 2

Fig. 3

16

# Fig.4

# Fig.5

# Fig.6

Fig. 7

4

Fig. 8

4

Fig. 9

4

18

Fig.10

4

Fig.11

4

Fig.12

4

Fig.13

12    13

4

13'

12'

## Fig-14

## Fig.15

Fig.16